# EUROPÄISCHE PATENTANMELDUNG

(11) **EP 2 228 658 A1**
(43) Veröffentlichungstag der Anmeldung: **15.09.2010**
(21) Anmeldenummer: 09155088.9
(22) Anmeldetag: 13.03.2009
(51) Int. Cl.: G01N 35/00

(54) **Verfahren zur Herstellung eines analytischen Verbrauchsmittels**

(71) Anmelder: Roche Diagnostics GmbH, 68305 Mannheim (DE); F. Hoffmann-La Roche AG, 4070 Basel (CH)
(72) Erfinder: Schwöbel, Wolfgang, 68309 Mannheim (DE); Dreibholz, Jörg, 67122 Altrip (DE)
(74) Vertreter: Stößel, Matthias

(57) **Zusammenfassung**

Es wird ein Verfahren zur Herstellung eines analytischen Verbrauchsmittels (112) vorgeschlagen. Das analytische Verbrauchsmittel (112) umfasst mindestens einen Träger (116) und mindestens ein mit dem Träger (116) verbundenes analytisches Hilfsmittel (118). Es wird mindestens ein optisch sensitives Material (128) auf den Träger (116) aufgebracht, welches eingerichtet ist, um bei Einwirkung einer elektromagnetischen Strahlung (146) mindestens eine optisch detektierbare Veränderung durchzuführen. In mindestens einem Codierungsschritt wird mindestens eine Funktionsinformation (150) über das analytische Verbrauchsmittel (112) in das optisch sensitive Material (128) mittels elektromagnetischer Strahlung (146) eingebracht. Die Funktionsinformation (150) ist eingerichtet, um mindestens einem analytischen Gerät einen korrekten Gebrauch des analytischen Verbrauchsmittels (112) zu ermöglichen.

## Beschreibung

### Gebiet der Erfindung

Die Erfindung betrifft ein Verfahren und eine Vorrichtung zur Herstellung eines analytischen Verbrauchsmittels. Derartige analytische Verbrauchsmittel werden insbesondere in der medizinischen Diagnostik eingesetzt, um qualitativ und/oder quantitativ mindestens einen Analyten in einer Probe nachzuweisen, beispielsweise einer Probe einer Körperflüssigkeit. Auch andere Anwendungsgebiete sind jedoch denkbar, beispielsweise in der chemischen Analytik.

### Stand der Technik

Insbesondere in der medizinischen Diagnostik sind zahlreiche Arten von Verbrauchsmitteln bekannt, welche üblicherweise als Einwegartikel ausgestaltet sind und welche schnell, zuverlässig und kostengünstig hergestellt werden müssen. So ermöglicht beispielsweise die Untersuchung von Blutproben oder anderer Proben von Körperflüssigkeiten, beispielsweise interstitieller Flüssigkeit, in der klinischen Diagnostik das frühzeitige und zuverlässige Erkennen von pathologischen Zuständen sowie eine gezielte und fundierte Kontrolle von Körperzuständen.

Die medizinische Diagnostik setzt in der Regel die Gewinnung einer Probe aus Blut oder interstitieller Flüssigkeit des zu untersuchenden Patienten voraus. Zu diesem Zweck wird üblicherweise die Haut, beispielsweise an der Fingerbeere oder dem Ohrläppchen, mit Hilfe einer sterilen, spitzen oder scharfen Lanzette perforiert, um so eine geringe Menge an Blut für die Analyse zu gewinnen. Die eigenhändige Blutzuckerbestimmung ist heute eine weltweit verbreitete Methode in der Diabetes-Kontrolle. Blutzuckergeräte im Stand der Technik weisen in der Regel ein Analysegerät auf, welches mit mindestens einem analytischen Verbrauchsmittel, insbesondere einem Testelement, zusammenwirkt. Die zu analysierende Probe wird dabei in der Regel auf ein Testfeld des Testelements aufgebracht und reagiert in dem Testfeld gegebenenfalls mit einem oder mehreren Reagenzien, welche in der Regel spezifisch für den nachzuweisenden Analyten ausgewählt sind. Diese Reaktion lässt sich nachweisen, beispielsweise auf optischem Wege und/oder auf elektrochemischem Wege.

Analytische Verbrauchsmittel in Form von Testelementen sind nur ein Ausführungsbeispiel einer Vielzahl von in der Analytik, insbesondere der medizinischen Analytik, verwendeten Verbrauchsmitteln. Zahlreiche weitere Anwendungen, welche Verbrauchsmittel einsetzen, sind denkbar. Grundsätzlich lässt sich die unten beschriebene Erfindung auf alle Arten derartiger analytischer Verbrauchsmittel gemäß dem Stand der Technik einsetzen.

Bei der Verwendung derartiger analytischer Verbrauchsmittel, insbesondere in der medizinischen Diagnostik, treten in der Praxis jedoch eine Reihe von technischen Herausforderungen auf, welche durch aufwendige apparative Lösungen überwunden werden müssen. So besteht eine Schwierigkeit darin, dass verschiedene Arten analytischer Verbrauchsmittel, welche in einem Analysesystem eingesetzt werden können, untereinander Unterschiede aufweisen können. So können sich beispielsweise Unterschiede hinsichtlich des Herstellers und/oder des Herstellungsverfahrens, hinsichtlich der verwendeten Nachweisreagenzien, hinsichtlich des nachzuweisenden Analyten, hinsichtlich des einzusetzenden Analyseverfahrens und/oder Analysesystems, hinsichtlich der Bedingungen, unter denen die Analyse durchgeführt werden soll, hinsichtlich der Parameter und/oder der Algorithmen für die Auswertung von Messungen, hinsichtlich der Chargennummern, hinsichtlich Chargenspezifischer Besonderheiten, hinsichtlich des Produktionsverfahrens, hinsichtlich der Anzahl von Analysezonen auf einem Testelement oder Ähnliches ergeben. Informationen über die genannten Unterschiede und/oder andere Arten von Unterschieden sollten also an ein Analysegerät, welches das analytische Hilfsmittel einsetzt, übermittelt werden. Auch bei analytischen Hilfsmitteln mit Lanzetten oder anderen Arten medizinischer Einwegartikel können sich derartige Artikel- spezifische Informationen ergeben, insbesondere Informationen hinsichtlich des Herstellers, der Art der Lanzette, der zu verwendenden Lanzettensysteme oder Ähnliches. Derartige Informationen, welche eingerichtet sind, um mindestens einem medizinischen Gerät einen korrekten Gebrauch des analytischen Verbrauchsmittels oder von Komponenten dieses analytischen Verbrauchsmittels zu ermöglichen, werden im Folgenden allgemein als Funktionsinformationen bezeichnet. Derartige Funktionsinformationen können beispielsweise die oben genannten Informationen und/oder weitere, im Folgenden genannte Informationen umfassen. Alternativ oder zusätzlich können auch andere Informationen umfasst sein. Funktionsinformationen können beispielsweise Verbrauchsmittel-spezifische Informationen und/oder Hilfsmittel-spezifische Informationen umfassen. Dabei bezieht sich der Ausdruck Verbrauchsmittel-spezifische Informationen auf Informationen zum analytischen Verbrauchsmittel als Ganzem, wohingegen sich Hilfsmittel-spezifische Informationen vorwiegend auf einzelne analytische Hilfsmittel beziehen, beispielsweise einzelne Testfelder und/oder Lanzetten, welche in dem analytischen Verbrauchsmittel enthalten sind.

In vielen Fällen ist es daher erforderlich, ein analytisches Verbrauchsmittel entsprechend zu codieren, das heißt mit einem lesbaren Code zu versehen, um, sobald dies erforderlich ist, diese Informationen entsprechend bereitstellen zu können. Ein wichtiges Anwendungsbeispiel besteht in einem automatischen Einlesen von Funktionsinformationen, beispielsweise von Verbrauchsmittel-spezifischen Informationen, durch ein Analysegerät, welches medizinische Einwegartikel, wie beispielsweise Teststreifen, Testbänder oder Lanzetten, verwenden soll. Da ein manuelles Eingeben und Auslesen derartiger Funktionsinformationen in der Regel für den Patienten unzumutbar oder sogar undurchfiihrbar ist, sind aus dem Stand der Technik verschiedene Verfahren und Systeme bekannt, bei welchen Funktionsinformationen Informationen automatisch eingelesen werden können. So sind beispielsweise Systeme bekannt, bei welchen derartige Funktionsinformationen über Hochfrequenzetiketten, separat in ein Messgerät einzuschiebende Datenträger (beispielsweise sogenannte ROM-Keys) oder ähnliche Codeträger übermittelt werden können. Derartige zusätzliche Codeträger verursachen jedoch als zusätzliche Komponenten Mehrkosten in der Herstellung, was in der Regel unerwünscht ist. Zudem ist in aller Regel eine Benutzeraktion erforderlich, beispielsweise ein Einschieben des ROM-Keys in ein Messgerät, was die Möglichkeit einer Fehlbedienung integrieren kann.

Auch Systeme, in welchen Verbrauchsmittel selbst codiert sind, sind aus dem Stand der Technik bekannt. DE 198 49 539 A1 beschreibt ein portables Blutzuckermessgerät für die Selbstkontrolle eines Diabetes-Patienten. Das Messgerät benutzt einen Teststreifen zur Bestimmung von Blutglucose, der als Band mit mehreren Messabschnitten auf einer Kassette aufgespult ist. Dabei wird unter anderem vorgeschlagen, die Produktionsqualität auf dem Testband zu codieren, um eine automatische Kalibrierung des Blutzuckermessgeräts auf die jeweilige Produktionscharge einer Bandkassette zu ermöglichen.

Aus EP 0 299 517 A2 ist eine Bandkassette für eine biochemische Analyse bekannt, welche einen langen Testfilm mit einem Nachweisreagenz aufweist. Unter anderem wird dabei vorgeschlagen, zu Beginn des Testbandes einen Codierungsbereich auf dem Testband vorzusehen, welcher mindestens eine Information umfasst. Dieser Codierungsbereich kann beispielsweise mit dem Detektor, welcher für die optische Messung verwendet wird, ausgelesen werden.

Bekannte Codierverfahren, wie beispielsweise das in EP 0 299 517 A2 beschriebene Codierverfahren weisen jedoch in der Praxis zahlreiche Nachteile und technische Herausforderungen auf. So sind derartige Codierverfahren beispielsweise vergleichsweise aufwendig, da diese eine strukturierte Aufbringung von Markierungssubstanzen erforderlich machen. Dies wiederum stellt, da diese Strukturierung mit hoher Auflösung erfolgen muss und gleichzeitig flexibel auf die einzuschreibenden Informationen angepasst werden können muss, technisch hohe Anforderungen an die Herstellung. Weiterhin ist zu berücksichtigen, dass der Codierungsprozess unter hohen Reinheitsbedingungen stattfinden muss, da analytische Hilfsmittel, welche in dem analytischen Verbrauchsmittel enthalten sind, nicht durch Codierungsmaterialien kontaminiert werden sollen.

### Aufgabe der Erfindung

Es ist daher eine Aufgabe der vorliegenden Erfindung, ein Verfahren und eine Vorrichtung zur Herstellung eines analytischen Verbrauchsmittels bereitzustellen, welche die Nachteile bekannter Verfahren vermeiden und dennoch eine Codierung mit hoher Informationsdichte ermöglichen.

### Beschreibung der Erfindung

Diese Aufgabe wird durch ein Verfahren und eine Vorrichtung mit dem Merkmal der unabhängigen Ansprüche gelöst. Vorteilhafte Weiterbildungen der Erfindung sind in den Unteransprüchen dargestellt, welche einzeln oder in Kombination realisiert werden können. Das vorgeschlagene Verfahren kann insbesondere unter Verwendung einer erfindungsgemäßen Vorrichtung durchgeführt werden, und die Vorrichtung kann eingerichtet sein, um erfindungsgemäßes Verfahren durchzuführen. Insofern kann bezüglich möglicher Ausgestaltungen der Vorrichtung auf die Beschreibung des Verfahrens verwiesen werden und umgekehrt.

Das vorgeschlagene Verfahren dient der Herstellung eines analytischen Verbrauchsmittels. Das analytische Verbrauchsmittel weist mindestens einen Träger und mindestens ein mit dem Träger verbundenes analytisches Hilfsmittel auf. Unter einem analytischen Verbrauchsmittel ist dabei allgemein ein Gegenstand zu verstehen, welcher für einen einmaligen Gebrauch oder für einen lediglich wenige Male umfassenden Gebrauch in der Analytik, beispielsweise in der chemischen und/oder biochemischen und/oder medizinischen Analytik, bestimmt ist. Ein besonderer Schwerpunkt liegt dabei auf der medizinischen Analytik und/oder Diagnostik, also dem Nachweis mindestens eines Analyten in einer Probe, insbesondere einer flüssigen Probe, beispielsweise einer Körperflüssigkeit. Der Nachweis kann dabei qualitativ und/oder quantitativ erfolgen und kann beispielsweise den Nachweis eines Metaboliten umfassen. Ein besonderer Schwerpunkt liegt auf dem Nachweis von Glucose in Blut (Blutglucose) und/oder in Urin und/oder in interstitieller Flüssigkeit. Auch andere Arten von Analyten lassen sich jedoch grundsätzlich nachweisen, so dass beispielsweise Cholesterinmessungen, Lactatmessungen, Coagulationsmessungen oder ähnliche in der medizinischen Diagnostik erforderliche Messungen durchgeführt werden können. Auch eine Messung in anderen Arten von Flüssigkeiten ist möglich. Grundsätzlich lässt sich die Erfindung auch auf andere Gebiete der Naturwissenschaften und/oder Technik übertragen, in welchen eine Analytik erforderlich ist.

Entsprechend dem geplanten Verwendungszweck kann auch das mindestens eine analytische Hilfsmittel ausgestaltet sein. Unter einem analytischen Hilfsmittel ist dementsprechend eine Vorrichtung zu verstehen, welche dem jeweiligen analytischen Zweck des Verbrauchsmittels dient und/oder diesen Zweck unterstützt. Diese Vorrichtung kann vollständig mit übrigen Bestandteilen des analytischen Verbrauchsmittels integriert sein, kann jedoch auch als separates Element in dem analytischen Verbrauchsmittel enthalten sein. Beispielsweise kann ein derartiger Zweck, dem das analytische Hilfsmittel dient, in der Bereitstellung einer zu analysierenden Probe und/oder in der Analyse der Probe selbst, insbesondere im Nachweis mindestens eines Analyten, liegen. Dementsprechend kann das analytische Hilfsmittel beispielsweise ein oder mehrere der folgenden analytischen Hilfsmittel umfassen: ein Testfeld zum Nachweis mindestens eines Analyten in einer Probe, insbesondere zum Nachweis eines Metaboliten in einer Körperflüssigkeit; eine Vorrichtung zum Erzeugen und/oder Bereitstellen einer flüssigen Probe, insbesondere einer Lanzette. Das Testfeld kann beispielsweise eine Nachweischemie umfassen, welche mindestens ein Nachweisreagenz aufweist, welches bei Anwesenheit des mindestens einen Analyten mindestens eine messbare Eigenschaft physikalischer und/oder chemischer Natur ändert. Beispielsweise kann das Nachweisreagenz eine entsprechende Analyt-spezifische Reaktion durchführen, welche auf optischem und/oder elektrochemischem Wege nachgewiesen werden kann. Bezüglich der möglichen Ausgestaltungen derartiger Testfelder und/oder Testchemien kann auf den Stand der Technik verwiesen werden.

Auch die Vorrichtung zum Erzeugen und/oder Bereitstellen einer flüssigen Probe kann auf verschiedene Weisen eingerichtet sein. So kann beispielsweise eine Lanzette vorgesehen sein, welche eine scharfkantige Schneidfläche und/oder eine Spitze aufweist, welche eingerichtet sind, um eine Hautpartie eines Benutzers zu perforieren. Auch komplexere Vorrichtungen sind jedoch möglich, beispielsweise Vorrichtungen, welche, neben einem Perforationszweck, gleichzeitig einem Transportzweck dienen, also beispielsweise zusätzlich mindestens eine Kapillare und/oder ein anderes Transportmittel aufweisen. Verschiedene Ausgestaltungen sind möglich.

In dem analytischen Verbrauchsmittel kann eine Art analytischer Hilfsmittel vorgesehen sein, oder es können auch mehrere unterschiedliche Arten analytischer Hilfsmittel kombiniert werden. So kann beispielsweise eine Mehrzahl von Lanzetten bereitgestellt werden, beispielsweise auf einem analytischen Band, oder es kann eine Mehrzahl von Testfeldern bereitgestellt werden, beispielsweise ebenfalls auf einem analytischen Band bzw. Testband, oder es kann, alternativ, auch eine Anordnung mit beispielsweise alternierenden Testfeldern und Lanzetten vorgesehen sein. Verschiedene Ausgestaltungen sind denkbar.

Das analytische Verbrauchsmittel kann auf verschiedene Weisen ausgestaltet sein und auf verschiedene Weisen das mindestens eine analytische Hilfsmittel, vorzugsweise mehrere analytische Hilfsmittel, umfassen. So kann beispielsweise ein analytisches Band mit einem Trägerband und einer Mehrzahl von auf dem Trägerband angeordneten analytischen Hilfsmitteln vorgesehen sein. Unter einem Trägerband kann dabei beispielsweise ein Kunststoffband, ein Papierband, ein Laminatband, ein Gewebeband, eine Gliederkette oder eine ähnliche, flexible und längliche Trägervorrichtung verstanden werden. Alternativ oder zusätzlich kann das analytische Verbrauchsmittel auch beispielsweise einen Teststreifen mit mindestens einem analytischen Hilfsmittel umfassen, insbesondere ein Einzeldisposable, beispielsweise ein Einzeldisposable mit einem oder mehreren Codierungsfeldern. Wiederum alternativ oder zusätzlich kann das analytische Verbrauchsmittel eine Testscheibe mit einer Mehrzahl von auf einer Oberfläche und/oder einer Kante der Testscheibe angeordneten analytischen Hilfsmitteln umfassen. Ebenfalls alternativ oder zusätzlich kann ein faltbares Verbrauchsmittel gegeben sein, mit einer Mehrzahl analytischer Hilfsmittel, beispielsweise nach einem Leporello-Prinzip.

Entsprechend dem mindestens einen analytischen Verbrauchsmittel und dem mindestens einen analytischen Hilfsmittel kann auch der Träger ausgestaltet sein. So kann der Träger beispielsweise flexibel und/oder verformbar ausgestaltet sein oder kann auch, je nach Einsatzzweck, steife Eigenschaften aufweisen. So ist beispielsweise im Falle eines Trägerbands und im Falle eines faltbaren Verbrauchsmittels die Verwendung eines verformbaren Trägers bevorzugt, wohingegen im Falle einer Testscheibe in der Regel starre Eigenschaften des Trägers gewünscht sind. Der Träger kann dementsprechend beispielsweise wiederum ein Papiermaterial, ein Kunststoffmaterial, ein Keramikmaterial, ein Gewebematerial oder eine Kombination der genannten und/oder anderer Materialien umfassen.

Ohne Beschränkung möglicher weiterer Ausgestaltungen der Erfindung wird die Erfindung im Folgenden im Wesentlichen in Bezugnahme auf ein analytisches Verbrauchsmittel in Form einer Bandkassette beschrieben, welche ein analytisches Band mit einem Trägerband und einer Mehrzahl von auf dem Trägerband angeordneten analytischen Hilfsmitteln umfasst. Insbesondere wiederum wird hier der Schwerpunkt auf analytische Hilfsmittel in Form von Testfeldern gelegt, wobei jedoch, wie oben beschrieben, allgemein auch andere Ausgestaltungen möglich sind.

Bei erfindungsgemäßen Herstellungsverfahren wird mindestens ein optisch sensitives Material auf den Träger aufgebracht. Das optisch sensitive Material ist eingerichtet, um bei Einwirkung einer elektromagnetischen Strahlung mindestens eine optisch detektierbare Veränderung durchzuführen. Das optisch sensitive Material sollte vorzugsweise nicht identisch mit dem Material des Trägers sein. Der Schritt des Aufbringens des optisch sensitiven Materials sollte also separat von der Herstellung des Trägers durchgeführt werden. Alternativ oder zusätzlich sind jedoch auch andere Ausgestaltungen grundsätzlich möglich, also Ausgestaltungen, bei welchen beispielsweise das optisch sensitive Material ganz oder teilweise identisch ist mit einem oder mehreren Materialien des Trägers. In diesem Fall kann der Schritt des Aufbringens des optisch sensitiven Materials auch ganz oder teilweise mit einer Herstellung des Trägers selbst zusammengefasst werden.

Die elektromagnetische Strahlung und damit die Sensitivität des optisch sensitiven Materials kann insbesondere im sichtbaren und/oder infraroten und/oder ultravioletten Spektralbereich liegen, so dass besonders bevorzugt eine Sensitivität für Licht besteht. Grundsätzlich lässt sich dabei ein optisch sensitives Material und/oder eine Kombination optisch sensitiver Materialien einsetzen, welche eine beliebige optisch detektierbare Veränderung durchführen. Dies kann beispielsweise eine Veränderung der Farbeigenschaften, eine Veränderung von Reflexionseigenschaften, eine Veränderung von Absorptionseigenschaften oder auch eine Veränderung eines Brechungsindex sein. Auch eine Kombination der genannten Eigenschaften und/oder anderer optisch detektierbarer Eigenschaften ist einsetzbar.

Bei dem erfindungsgemäßen Verfahren wird weiterhin in mindestens einem Codierungsschritt mindestens eine Funktionsinformation über das analytische Verbrauchsmittel in das optisch sensitive Material mittels elektromagnetischer Strahlung eingeschrieben. Die Funktionsinformation ist eingerichtet, um mindestens einem analytischen Gerät, welches mit dem analytischen Verbrauchsmittel zusammenwirkt, einen korrekten Gebrauch des analytischen Verbrauchsmittels zu ermöglichen. Das analytische Gerät kann beispielsweise ein medizinisches Gerät umfassen, beispielsweise ein medizinisches Handgerät, insbesondere ein Handgerät, welches für die medizinische Diagnostik eingesetzt werden kann. Beispielsweise kann es sich dabei um ein Blutzuckermessgerät mit mindestens einer Messfunktion und/oder mindestens einer Probengewinnungsfunktion handeln, also beispielsweise der Messung eines Blutglucosegehalts in einer Blutprobe und/oder der Generierung der Blutprobe. Auch andere Ausgestaltungen sind jedoch denkbar.

Unter einer Funktionsinformation wird dabei allgemein eine Information verstanden, welche das Zusammenwirken des analytischen Verbrauchsmittels mit dem analytischen Gerät betrifft, d.h. dieses Zusammenwirken ermöglicht oder unterstützt. Für mögliche Inhalte oder Ausgestaltungen der Funktionsinformation kann beispielsweise auf die obige Beschreibung des Standes der Technik verwiesen werden. Von diesem Begriff nicht umfasst sein sollen Informationen über eine Fehlerhaftigkeit des analytischen Verbrauchsmittels, also Fehlerinformationen, beispielsweise eine Information über eine Fehlerhaftigkeit, eine Fehlerfreiheit, einen Qualitätsgrad oder ähnliche Fehlerinformationen. Derartige Fehlerinformationen können jedoch zusätzlich zu der mindestens einen Funktionsinformation umfasst sein.

Dabei wird im Folgenden zwischen der mindestens einen Funktionsinformation und deren Bedeutungsinhalt und/oder physikalischer Form begrifflich nicht unterschieden. So können Funktionsinformationen beispielsweise in für einen Menschen wahrnehmbarer und entschlüsselbarer Form vorliegen, beispielsweise als alphanumerische Zeichen. Alternativ oder zusätzlich können die Funktionsinformationen jedoch auch codiert vorliegen, so dass deren Informationsinhalt erst nach einem entsprechenden Decodierungsschritt wahrnehmbar und/oder verwendbar ist. Letzteres wird auch als Code bezeichnet, wobei wiederum beispielsweise zwischen für einen Menschen lesbaren Codes und lediglich maschinenlesbaren Codes unterschieden werden kann.

Dementsprechend kann die mindestens eine Funktionsinformation beispielsweise, wie oben ausgeführt, mindestens eine Verbrauchsmittel-spezifische und/oder Hilfsmittel-spezifische Information umfassen. Beispielsweise kann die mindestens eine Funktionsinformation eine oder mehrere der folgenden Informationen umfassen: eine Information über einen Hersteller und/oder ein Herstellungsverfahren; eine Information über ein enthaltenes Nachweisreagens; eine Information hinsichtlich eines nachzuweisenden Analyten; eine Information hinsichtlich eines einzusetzenden Analyseverfahrens und/oder Analysesystems; eine Information hinsichtlich der Bedingungen, unter denen eine Analyse durchgeführt werden soll; eine Information hinsichtlich von Parametern und/oder Algorithmen für eine Auswertung von Messungen, insbesondere mindestens einen Korrekturfaktor und/oder mindestens eine Funktionskurve; eine Information hinsichtlich von Chargennummern und/oder mindestens eine Individualkennzeichnung; eine Information hinsichtlich chargenspezifischer Besonderheiten; eine Information hinsichtlich einer Anzahl von analytischen Hilfsmitteln; eine Information hinsichtlich einer Art einer Vorrichtung zum Erzeugen und/oder Bereitstellen einer flüssigen Probe, insbesondere einer Lanzette; eine Information hinsichtlich einer zu verwendenden Vorrichtung zum Erzeugen und/oder Bereitstellen einer flüssigen Probe, insbesondere einer Lanzette; ein Haltbarkeitsinformation, insbesondere ein Haltbarkeitsdatum und/oder eine Haltbarkeitsbeschränkung; eine Verwendungsbeschränkung. Eine Verwendungsbeschränkung kann beispielsweise bestimmte Verwendungen des analytischen Hilfsmittels in einem analytischen Gerät einschränken, wenn das analytische Gerät diese Verwendungsbeschränkung liest. Beispielsweise kann die Verwendungsbeschränkung in dem analytischen Gerät dazu genutzt werden, dass ein bestimmtes analytische Verbrauchsmittel oder ein Teil davon auf diesem Gerät nicht verwendet werden darf oder zu ähnlichen Zwecken. Beispielsweise kann eine Verwendungsbeschränkung eine Sperrinformation umfassen, welche die Verwendung auf einem bestimmten Gerät sperrt.

Insbesondere kann eine Information über einen Hersteller und/oder ein Herstellungsverfahren umfasst sein. Dieser Hersteller bzw. das Herstellungsverfahren kann das analytische Verbrauchsmittel als Ganzes betreffen oder auch Teile desselben, beispielsweise ein oder mehrere in dem analytischen Verbrauchsmittel umfasste analytische Hilfsmittel. Alternativ oder zusätzlich kann die Funktionsinformation eine Information über ein enthaltenes Nachweisreagenz umfassen. Beispielsweise können eine Art des Nachweisreagenz in codierter Form enthalten sein, ein Verfallsdatum, ein Herstellungsdatum, eine chargenspezifische Sensitivität oder Ähnliches. Wiederum alternativ oder zusätzlich kann eine Information hinsichtlich eines nachzuweisenden Analyten umfasst sein. Ebenfalls alternativ oder zusätzlich kann eine Information hinsichtlich eines einzusetzenden Analyseverfahrens und/oder Analysesystems umfasst sein. Beispielsweise kann in codierter Form beinhaltet sein, welches Blutglucosemessgerät für das jeweilige analytische Verbrauchsmittel und/oder ein oder mehrere in diesem enthaltene analytische Hilfsmittel geeignet sind. Auf diese Weise lassen sich beispielsweise Bedienungsfehler rechtzeitig erkennen, indem beispielsweise ausgeschlossen wird, dass ein ungeeignetes analytisches Gerät für das jeweilige Verbrauchsmittel eingesetzt wird oder umgekehrt. Weiterhin kann, ebenfalls wiederum alternativ oder zusätzlich, eine Information hinsichtlich der Bedingungen, unter denen eine Analyse durchgeführt werden soll, umfasst werden. Beispielsweise können bei optischen Testelementen Parameter einer optimalen Beleuchtung vorgegeben werden und/oder es können bei elektrochemischen Testelementen optimale Strom- und/oder Spannungsparameter vorgegeben werden. Verschiedene andere Ausgestaltungen sind denkbar. Wiederum alternativ oder zusätzlich kann eine Information hinsichtlich von Parametern und/oder Algorithmen für eine Auswertung von Messungen vorgegeben werden. Insbesondere lassen sich auf diese Weise beispielsweise Korrekturfaktoren und/oder mindestens eine Funktionskurve vorgeben, welche für die Auswertung einer Analyse erforderlich sein kann. Auf diese Weise lassen sich insbesondere chargenspezifische Schwankungen ausgleichen, indem Korrekturfaktoren und/oder Funktionskurven für jede Charge von Verbrauchsmittel und/oder für jedes einzelne analytische Hilfsmittel in dem Verbrauchsmittel mitgeliefert werden. Eine separate Eingabe derartiger chargenspezifischer Besonderheiten durch einen Benutzer und/oder durch einen separaten Datenträger ist nicht erforderlich. Wiederum alternativ oder zusätzlich kann die Funktionsinformation beispielsweise eine Funktion hinsichtlich einer Anzahl von analytischen Hilfsmitteln umfassen. So kann beispielsweise eine Gesamtanzahl von analytischen Hilfsmitteln in dem Verbrauchsmittel angegeben werden und/oder eine Anzahl von noch zur Verfügung stehenden und/oder bereits verbrauchten analytischen Hilfsmitteln. Diese Information kann beispielsweise von dem analytischen Gerät dazu genutzt werden, um einen Benutzer rechtzeitig zur Anschaffung und/oder Bereitstellung eines neuen analytischen Verbrauchsmittels aufzufordern. Wiederum alternativ oder zusätzlich kann eine Information hinsichtlich einer Art einer Vorrichtung zum Erzeugen und/oder Bereitstellen einer flüssigen Probe umfasst sein. Wie oben dargestellt, kann es sich bei dieser Vorrichtung insbesondere um eine Lanzette handeln. Die Information kann beispielsweise genutzt werden, um ein korrektes Antriebssystem für die jeweilige Lanzette einzusetzen und/oder die Verwendung ungeeigneter Lanzetten durch das analytische Gerät auszuschließen. Wiederum alternativ oder zusätzlich kann auch eine Information hinsichtlich einer zu verwendenden Vorrichtung zum Erzeugen und/oder Bereitstellen einer flüssigen Probe, insbesondere einer Lanzette, umfasst sein, so dass ein Benutzer beispielsweise von vorne herein aufgefordert werden kann, eine korrekte Vorrichtung und/oder ein korrektes analytisches Verbrauchsmittel in das analytische Gerät einzusetzen.

Im Unterschied zu den bekannten Codierungsverfahren, wie beispielsweise dem in EP 0 299 517 A2 offenbarten Codierungsverfahren, lässt sich das vorgeschlagene Codierungsverfahren der vorliegenden Erfindung vergleichsweise einfach ausgestalten, wobei dennoch Codierungen mit hoher Informationsdichte aufgebracht werden können. Im Gegensatz zu den bekannten Druckverfahren zum Aufbringen von Farbmarkierungen, Barcodes oder Ähnlichem kann die Aufbringung des optisch sensitiven Materials auf den Träger vergleichsweise grob, unstrukturiert oder lediglich mit geringer Auflösung strukturiert erfolgen. Es lässt sich dabei auf aufwendige Druckverfahren vollständig verzichten. Die eigentliche Codierung, welche mit hoher Auflösung erfolgen kann, kann anschließend strukturiert durch die elektromagnetische Strahlung erfolgen, welche mit hoher Energiedichte eingesetzt werden kann.

Dabei lassen sich durch Verwendung geeigneter gebündelter und/oder fokussierter Strahlen hohe Auflösungen erzielen, wofür bei dem erfindungsgemäßen Verfahren beispielsweise eine entsprechende Optik bereitgestellt werden kann. Dabei können eine oder mehrere elektromagnetische Strahlenquellen vorgesehen sein, welche geeignete elektromagnetische Strahlung bereitstellen können. Grundsätzlich lassen sich beliebige Arten derartiger elektromagnetischer Strahlenquellen verwenden. Besonders bevorzugt aufgrund der möglichen hohen Energiedichte und der möglichen starken Bündelung und/oder Fokussierung ist jedoch die Verwendung eines oder mehrerer Laser, so dass die elektromagnetische Strahlung vorzugsweise mindestens eine Laserstrahlung umfasst. Dabei können beispielsweise Laser im ultravioletten Spektralbereich und/oder im sichtbaren Spektralbereich und/oder im infraroten Spektralbereich eingesetzt werden. Da Laserstrahlung üblicherweise eine sehr schmalbandige Strahlung bereitstellt, kann mittels einer geeigneten Wahl der Wellenlänge der Laserstrahlung gezielt eine Anpassung der elektromagnetischen Strahlung auf die Eigenschaften des optisch sensitiven Materials erfolgen. So kann beispielsweise eine elektromagnetische Strahlung gewählt werden, welche in einem Wellenlängenbereich liegt, in welchem das optisch sensitive Material eine hohe Absorption, beispielsweise einen Absorptionspeak, aufweist. Durch diese gezielte Wahl der Wellenlänge kann auch eine Beschädigung weiterer Komponenten des analytischen Verbrauchsmittels, wie beispielsweise des Trägers, vermieden werden, indem beispielsweise die Wahl der Wellenlänge derart erfolgt, dass der Träger in dem Bereich dieser Wellenlänge nicht oder nur geringfügig absorbiert, wohingegen das optisch sensitive Material in höherem Maße absorbiert. So kann beispielsweise die Absorption, beispielsweise ein Absorptionskoeffizient, des optisch sensitiven Materials bei der gewählten Wellenlänge und/oder in dem gewählten Wellenlängenbereich der elektromagnetischen Strahlung um mindestens einen Faktor zwei, vorzugsweise um mindestens einen Faktor zehn, hundert oder mehr, höher ausgestaltet sein als die Absorption des Trägers.

Trotz der Möglichkeit, das optisch sensitive Material unstrukturiert oder lediglich mit geringer Auflösung strukturiert auf den Träger aufzubringen, beispielsweise in Form von einem oder mehreren Codierungsfeldern mit Abmessungen im Bereich von mindestens 500 µm, vorzugsweise von mindestens 1 mm, lässt sich mittels der elektromagnetischen Strahlung eine hohe Informationsdichte erzielen. So lassen sich fein strukturiert eindimensionale, zweidimensionale oder dreidimensionale Strukturen in das optisch sensitive Material einbringen, insbesondere einschreiben. Beispielsweise lässt sich in dem Codierungsschritt mindestens ein eindimensionaler und/oder zweidimensionaler und/oder mindestens ein dreidimensionaler Barcode in das optisch sensitive Material einbringen. Beispielsweise kann es sich dabei um normierte Barcodes handeln, welche mit üblichen Barcodelesern und/oder unter Verwendung üblicher Decodierungsalgorithmen für Barcodes ausgewertet werden können, um die darin enthaltene Funktionsinformation zu entschlüsseln. Unter einem Barcode kann dabei jedoch grundsätzlich eine beliebige Markierung verstanden werden, welche als Informationsträger dienen kann, insbesondere als optischer Informationsträger, beispielsweise als Informationsträger mit mehreren Informationsfeldern.

Allgemein lassen sich zum Auslesen der mindestens einen Funktionsinformation beliebige Detektoren einsetzen, welche ganz oder teilweise in dem analytischen Gerät enthalten sein können. Dabei können zusätzliche Detektoren eingesetzt werden, welche unabhängig von der übrigen Funktionalität des analytischen Geräts sind. Alternativ oder zusätzlich lassen sich jedoch auch Detektoren einsetzen, welche ohnehin in dem analytischen Gerät vorhanden sein können, indem diese Detektoren in einer Mehrfachfunktion eingesetzt werden können. Wird beispielsweise ein analytisches Gerät in Form eines optischen Messgeräts, beispielsweise eines Blutglucosemessgeräts auf optischer Basis, eingesetzt, so ist in der Regel mindestens ein optischer Detektor in diesem analytischen Gerät vorhanden, welcher beispielsweise Farben oder Farbänderungen in einem oder mehreren Testfeldern des analytischen Verbrauchsmittels registriert. Dieser Detektor kann in einer Mehrfachfunktion eingesetzt werden, um zusätzlich auch die mindestens eine Funktionsinformation auszulesen. So können zum Lesen der Funktionsinformation, welche in codierter Form vorliegen kann, beispielsweise ein geräteseitig vorhandener Positionssensor, eine Messoptik oder ein zusätzlicher Sensor verwendet werden. Die mindestens eine Funktionsinformation kann beispielsweise in einem oder mehreren Codierungsfeldern vorgesehen sein. Diese Codierungsfelder können beispielsweise auf einem Band durch ein vorhandenes Positionssensorfenster in einem Gehäuse einer Bandkassette, durch ein zusätzliches Fenster und/oder durch ein transparentes Gehäuse, beispielsweise ein Kunststoffgehäuse, hindurch zugänglich sein.

Das analytische Verbrauchsmittel kann dementsprechend mindestens ein Gehäuse aufweisen, in welchem das analytische Hilfsmittel zumindest teilweise aufgenommen ist. Dabei kann das Gehäuse mindestens einen transparenten Bereich, insbesondere ein Fenster, aufweisen, wobei der transparente Bereich für die elektromagnetische Strahlung, beispielsweise die Laserstrahlung, zumindest teilweise transparent sein kann. Alternativ oder zusätzlich kann dieser mindestens eine transparente Bereich auch für einen optischen Nachweis der optisch detektierbaren Veränderung zumindest teilweise transparent sein, also beispielsweise für eine bei diesem Nachweis verwendete mindestens eine Detektionswellenlänge und/oder einen Detektionswellenlängenbereich. Auf diese Weise kann beispielsweise ein gedrucktes Codierungsfeld auf einem Band durch ein Positionssensorfenster, durch ein zusätzliches Fenster oder durch ein für den Laser bzw. eine Detektionswellenlänge durchlässiges Kunststoffgehäuse hindurch beschrieben und/oder gelesen werden.

Werden Sensoren in dem analytischen Gerät in einer Mehrfachfunktion verwendet, so können beispielsweise ein Bandsensor und/oder eine Messoptik eingesetzt werden, welche die Fähigkeit aufweisen, durch das Fenster hindurch für die Detektion einer Bandposition bzw. durch ein Messfenster zur Auswertung eines Testfeldes bzw. einer Testchemie hindurch auch die mindestens eine Funktionsinformation, beispielsweise einen Strichcode mit einer Chargenkalibrationsinformation, zu lesen.

Wie oben dargestellt, kann das optisch sensitive Material beispielsweise in Form mindestens eines Codierungsfeldes auf den Träger aufgebracht werden. Dabei lassen sich beispielsweise polygonale, insbesondere rechteckige, und/oder runde, beispielsweise kreisförmige und/oder ovale, Codierungsfelder einsetzen. Vorteilhaft an dem vorgeschlagenen Verfahren ist dabei, dass diese Codierungsfelder, wie oben dargestellt, mit geringer Auflösung aufgebracht werden können. So können beispielsweise die Codierungsfelder laterale Abmessungen, beispielsweise Durchmesser und/oder Kantenlängen, aufweisen, welche mindestens 200 µm, insbesondere mindestens 500 µm und vorzugsweise mindestens 1 mm, betragen. Die eigentliche, hochauflösend einbringbare Funktionsinformation wird dann mittels der elektromagnetischen Strahlung eingebracht, was, wie oben beschrieben, ohne hohen technischen Aufwand mit hoher Auflösung erfolgen kann. Insofern kann eine hohe Informationsdichte erzielt werden.

Zum Aufbringen des Codierungsfeldes können beispielsweise Druckverfahren eingesetzt werden, bei welchen das optisch sensitive Material und/oder ein Vorstoff des optisch sensitiven Materials auf den Träger aufgedruckt werden. Unter einem Vorstoff sind dabei Materialien zu verstehen, welche nach mindestens einem Umwandlungsschritt das optisch sensitive Material bilden. Bei diesem Umwandlungsschritt kann es sich um einen beliebigen physikalischen und/oder chemischen Schritt handeln, beispielsweise eine Reaktion und/oder eine Phasenumwandlung und/oder eine Trocknung und/oder eine Vernetzung. Alternativ oder zusätzlich lassen sich Laminierverfahren einsetzen, bei welchen mindestens ein Film des optisch sensitiven Materials auf den Träger auflaminiert und/oder aufgeklebt wird. Auf diese Weise lassen sich die Codierungsfelder beispielsweise vorfertigen, um diese dann als ganze auf den Träger aufzubringen. Beispielsweise lassen sich zu diesem Zweck herkömmliche Etikettierverfahren einsetzen.

Das mindestens eine Codierungsfeld ist vorzugsweise räumlich getrennt von dem mindestens einen analytischen Hilfsmittel angeordnet. Sind eine Mehrzahl analytischer Hilfsmittel vorgesehen, so können jeweils einem einzelnen analytischen Hilfsmittel, einer Gruppe analytischer Hilfsmittel oder allen analytischen Hilfsmitteln ein oder mehrere spezifische Codierungsfelder zugeordnet sein. Beispielsweise kann ein Band vorgesehen sein, auf welchem mehrere analytische Hilfsmittel angeordnet sind. In diesem Fall kann beispielsweise alternierend vor und/oder nach jedem analytischen Hilfsmittel, jedem zweiten analytischen Hilfsmittel oder jedem n-ten analytischen Hilfsmittel mindestens ein Codierungsfeld alternierend zu den analytischen Hilfsmitteln angeordnet sein, welches die Funktionsinformation für das mindestens eine analytische Hilfsmittel spezifisch trägt. Auch andere andere Ausgestaltungen sind jedoch möglich.

Das mindestens eine Codierungsfeld kann auch weitere Funktionen übernehmen. So kann dieses mindestens eine Codierungsfeld beispielsweise weiterhin die Funktion einer Positionsmarkierung übernehmen und/oder mit einem oder mehreren Positionsmarkern auf dem analytischen Verbrauchsmittel zusammengefasst sein. Beispielsweise können Codierungsfelder an mehreren vorgegebenen und/oder bekannten Positionen auf dem analytischen Verbrauchsmittel angeordnet werden, wobei die Codierungsfelder eingerichtet sind, um ganz oder teilweise als Positionsmarkierungen verwendet zu werden. Diese Einrichtung kann beispielsweise dadurch erfolgen, dass diese an bekannten Positionen angeordnet sind. Alternativ oder zusätzlich können die Codierungsfelder auch eine geometrische Form aufweisen, welche von dem analytischen Gerät auf einfache Weise erkannt werden kann, um auf diese Weise eine Positionsmarkierung bzw. eine Positionsbestimmung durchzuführen. Eine derartige Positionsmarkierung ist insbesondere bei Bandkassetten von Vorteil. Wie oben dargestellt, kann das optisch sensitive Material auf verschiedene Weise ausgestaltet sein. So kann beispielsweise dieses optisch sensitive Material als zusätzliches Farbfeld als Codeträger für die Funktionsinformation auf den Träger, beispielsweise ein Reagenzträgerband, aufgedruckt werden. Das optisch sensitive Material kann beispielsweise als laseraktivierbares Material ausgeführt sein. Entsprechend kann das mindestens eine Codierungsfeld beispielsweise als laseraktivierbares Feld ausgeführt sein.
Wie oben beschrieben, wird in dem Codierungsschritt lokal eine optisch detektierbare Änderung des mindestens einen optisch sensitiven Materials durchgeführt. Dementsprechend kann das mindestens eine optisch sensitive Material eine Vielzahl bekannter Materialien umfassen, welche derartige optisch detektierbare Veränderungen bei Einwirkung mindestens einer elektromagnetischen Strahlung durchlaufen. Beispielsweise kann es sich bei diesen Materialien um Farbstoffe handeln, beispielsweise Farbstoffe, die bei Einwirkung der elektromagnetischen Strahlung ausbleichen und/oder einen Farbumschlag durchführen, insbesondere auch einen Umschlag von Schwarz zu Weiß oder umgekehrt, welche zu einer Fluoreszenz und/oder Phosphoreszenz angeregt werden oder welche auch ähnliche Weise ihre Absorptions- und/oder Transmissionseigenschaften verändern. Der Farbstoff kann dabei als Einzelschicht verwendet werden, kann jedoch insbesondere auch in einem Matrixmaterial gelöst und/oder dispergiert oder auf andere Weise eingebettet sein. Beispielsweise können laseraktivierbare Farbstoffe eingesetzt werden. Weiterhin können auch Pigmente verwendet werden, also anorganische oder organische, bunte oder unbunte Farbmittel, welche nicht in gelöster Form vorliegen. Beispielsweise kann es sich dabei um Laseraktivierbare Pigmente handeln. Ebenfalls alternativ oder zusätzlich kann, wie oben dargestellt, die optische detektierbare Veränderung auch beispielsweise eine Veränderung eines Brechungsindex umfassen. Dabei können beispielsweise Techniken eingesetzt werden, welche typischerweise in der holographischen Datenspeicherung verwendet werden, so dass in das optisch sensitive Material beispielsweise ein derartiges Hologramm eingebracht werden kann. Beispielsweise lassen sich zu diesem Zweck Kunststoffmaterialien verwenden. Insbesondere können hier Kunststoffe eingesetzt werden, welche bei Einwirkung der elektromagnetischen Strahlung einen Brechungsindex verändern. So kann, alternativ oder zusätzlich zu beispielsweise einem laseraktivierbaren Druck, welcher einen Schwarz-Weiß-Umschlag ermöglicht, auch ein geeignetes Folienmaterial optisch erkennbar strukturiert werden, beispielsweise ähnlich zu bekannten, in einen Klebefilm einbrennbaren Hologrammen. Mittels geeigneter Sensoren, beispielsweise ebenfalls Lasersensoren, ähnlich zur holographischen Datenspeicherung, kann die damit erzielte optisch detektierbare Veränderung erkannt und damit die mindestens eine Funktionsinformation wieder ausgelesen werden. Verschiedene Ausgestaltungen sind denkbar.

Besonders bevorzugt erfolgt der Codierungsschritt als abschließender oder näherungsweise das Herstellungsverfahren abschließender Verfahrensschritt. Besonders bevorzugt ist es, wenn das Verfahren weiterhin mindestens einen Kalibrationsschritt umfasst, wobei in dem Kalibrationsschritt mindestens eine Eigenschaft mindestens eines in dem analytischen Verbrauchsmittel umfassten analytischen Hilfsmittels gemessen wird. Diese mindestens eine Eigenschaft kann dann ganz oder teilweise als Bestandteil der mindestens einen Funktionsinformation verwendet werden. Beispielsweise können Kalibrationsmessungen an einem oder mehreren Testfeldern durchgeführt werden, um Herstellungs-spezifische und/oder chargenspezifische Unterschiede zu ermitteln. Auf diese Weise lassen sich beispielsweise messtechnisch Informationen über die Parameter und/oder Algorithmen für eine Auswertung von Messungen bestimmen, beispielsweise Korrekturfaktoren und/oder Funktionskurven. Auch andere Arten von Eigenschaften können, alternativ oder zusätzlich, in dem Kalibrationsschritt ermittelt werden. Zur Durchführung des Kalibrationsschritts können bekannte Detektionsverfahren eingesetzt werden. Anschließend wird diese ermittelte Eigenschaft vorzugsweise zumindest teilweise in die Funktionsinformation oder in einen Teil dieser Funktionsinformation umgewandelt, und es wird anschließend der oben beschriebene Codierungsschritt durchgeführt. Auf diese Weise lassen sich die individuellen Eigenschaften der Verbrauchsmittel und/oder einzelner oder mehrerer in den Verbrauchsmitteln enthaltener analytischer Hilfsmittel gezielt bestimmen, so dass beispielsweise analytische Messungen, welche mit derartigen Verbrauchsmitteln durchgeführt werden, mit hoher Präzision und unter nahezu vollständigem Ausschluss von Verbrauchsmittel-bedingten Schwankungen durchgeführt werden können.

Beispielsweise kann zunächst bei der Herstellung ein zusätzliches Codierungsfeld, beispielsweise ein Farbfeld, als Codeträger auf ein Band, beispielsweise ein Reagenzträgerband, aufgedruckt werden, welches beispielsweise als laseraktivierbares Feld ausgeführt ist. Nachdem ansonsten das Verbrauchsmittel zumindest weitgehend fertiggestellt ist, kann im Kalibrationsschritt die mindestens eine Eigenschaft ermittelt werden, um diese dann in die mindestens eine Funktionsinformation umzuwandeln und auf das Codierungsfeld aufzubringen. So kann nach einer Chargenkalibration durch einen Laserprozess beispielsweise ein Strichcode in einem Codierungsfeld, beispielsweise einem pigmentierten Feld, erzeugt werden, welcher beispielsweise einen erforderlichen Chargencode umfassen kann.

Ein wesentlicher Vorteil der nachgeschalteten Codierung, im Gegensatz zu einer Codierung, bei welcher unmittelbar auf das Band bereits eine Kalibrationsinformation aufgedruckt wird, liegt also darin, dass die die Codierung zu einem späten, insbesondere spätestmöglichen, Zeitpunkt erfolgen kann. Der Kalibrationsschritt kann in einem Zustand durchgeführt werden, in welchem nur noch unwesentlich auf das analytische Verbrauchsmittel eingewirkt werden muss, so dass eine Veränderung der Eigenschaften des analytischen Verbrauchsmittels und/oder einzelner analytischer Hilfsmittel in dem Verbrauchsmittel zumindest weitgehend vermieden werden kann. Durch den Kalibrationsschritt und die nachfolgende Codierung in dem Codierungsschritt wird somit das analytische Verbrauchsmittel selbst in seinen Eigenschaften nur noch unwesentlich beeinflusst. Dieser Aspekt der Erfindung ist dementsprechend besonders vorteilhaft in Zusammenhang mit der oben beschriebenen Ausgestaltung der Erfindung, bei welcher das analytische Verbrauchsmittel ein Gehäuse mit mindestens einem transparenten Bereich aufweist, durch welchen beispielsweise die Codierung mittels der elektromagnetischen Strahlung erfolgen kann. Die Transparenz kann beispielsweise auch zumindest speziell in dem Bereich der für die Codierung und/oder für den optischen Nachweis der optisch detektierbaren Veränderung zur Anwendung kommende Wellenlänge ausgestaltet sein. In anderen Wellenlängenbereichen kann beispielsweise eine geringere Transparenz oder eine Intransparenz bestehen. Im Gegensatz hierzu wäre bei bekannten Codierungsverfahren mittels eines Druckprozesses eine vollständige Öffnung des Gehäuses erforderlich, was jedoch wiederum zu einer Kontamination einzelner oder mehrerer analytischer Hilfsmittel führen könnten.

Wie oben dargestellt, betrifft die Erfindung in einem weiteren Aspekt eine Vorrichtung zur Herstellung eines analytischen Verbrauchsmittels, welche insbesondere eingerichtet sein kann, um ein Verfahren gemäß einer oder mehrerer der oben beschriebenen Ausführungsformen durchzuführen. Dabei wird ein analytisches Verbrauchsmittel gemäß der obigen Beschreibung hergestellt, welches mindestens einen Träger und mindestens ein mit dem Träger verbundenes analytisches Hilfsmittel umfasst. Die Vorrichtung umfasst mindestens eine Aufbringvorrichtung zum Aufbringen eines optisch sensitiven Materials auf den Träger, wobei das optisch sensitive Material eingerichtet ist, um bei Einwirkung einer elektromagnetischen Strahlung mindestens eine optisch detektierbare Veränderung durchzuführen. Die Vorrichtung umfasst weiterhin mindestens eine Codiervorrichtung, welche eingerichtet ist, um mittels elektromagnetischer Strahlung mindestens eine Funktionsinformation über das analytische Verbrauchsmittel in das optisch sensitive Material einzubringen. Die Funktionsinformation ist, wie oben dargestellt, eingerichtet, um mindestens einem analytischen Gerät einen korrekten Gebrauch des analytischen Verbrauchsmittels zu ermöglich.

Für die möglichen Ausgestaltungen der einzelnen Komponenten der Vorrichtung kann beispielsweise auf die obige Beschreibung verwiesen werden. Die Codiervorrichtung kann beispielsweise mindestens eine Datenverarbeitungsvorrichtung umfassen, welche programmtechnisch eingerichtet sein kann, um die mindestens eine Funktionsinformation bereitzustellen. Dabei kann die Codiervorrichtung beispielsweise einen Dateneingang umfassen, über welchen die mindestens eine erforderliche Information, beispielsweise über eine Eigenschaft mindestens eines analytischen Hilfsmittels empfangen werden kann. Diese mindestens eine Information wird dann zu der Funktionsinformation umgewandelt bzw. es wird eine Funktionsinformation generiert, welche diese Information umfasst. Zu diesem Zweck kann beispielsweise die Datenverarbeitungsvorrichtung entsprechend programmtechnisch eingerichtet sein. Weiterhin kann die Codiervorrichtung, wie oben beschrieben, die mindestens eine Strahlenquelle zur Erzeugung der elektromagnetischen Strahlung, beispielsweise mindestens einen Laser, umfassen.

Für die Durchführung der weiteren oben beschriebenen Verfahrensschritte kann die Vorrichtung entsprechende Einzelvorrichtungen bereitstellen, welche zur Durchführung der genannten Schritte eingerichtet sind.

### Ausführungsbeispiele

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: ein schematisches Ausführungsbeispiel einer Vorrichtung zur Herstellung eines analytischen Verbrauchsmittels;
- Figur 2: ein Ausführungsbeispiel eines Analysebands als Teil eines analytischen Verbrauchsmittels; und
- Figur 3: ein Ausführungsbeispiel eines Codierungsschritts mit einer Lasermarkierung durch ein Gehäuse hindurch.

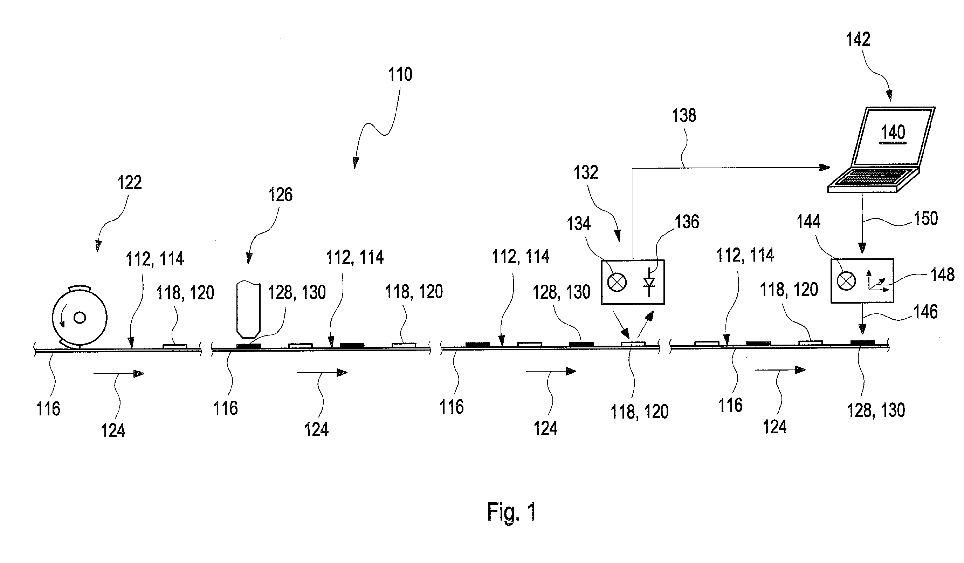

In Figur 1 ist ein Ausführungsbeispiel einer erfindungsgemäßen Vorrichtung 110 zur Herstellung eines analytischen Verbrauchsmittels 112 dargestellt. Anhand dieser Vorrichtung 110 soll auch ein mögliches Ausführungsbeispiel eines erfindungsgemäßen Verfahrens zur Herstellung des analytischen Verbrauchsmittels 112 erläutert werden. Es wird darauf hingewiesen, dass die Vorrichtung 110 auch zusätzliche, in Figur 1 nicht dargestellte Komponenten umfassen kann, entsprechend zu zusätzlichen möglichen Verfahrensschritten. Auch ist die in Figur 1 angedeutete Reihenfolge der Verfahrensschritte nicht notwendigerweise erforderlich, so dass auch Verfahrensschritte in ihrer Reihenfolge vertauscht werden können, seitlich parallel durchgefiihrt werden können oder wiederholt durchgeführt werden können. Dabei ist in Figur 1 ein In-Line-Prozess beschrieben, bei welchem die Verfahrensschritte nacheinander durchgeführt werden. Alternativ oder zusätzlich könnten jedoch auch einer oder mehrere der Verfahrensschritte zeitlich parallel durchgefiihrt werden, beispielsweise auf unabhängig voneinander operierenden Teilvorrichtungen.

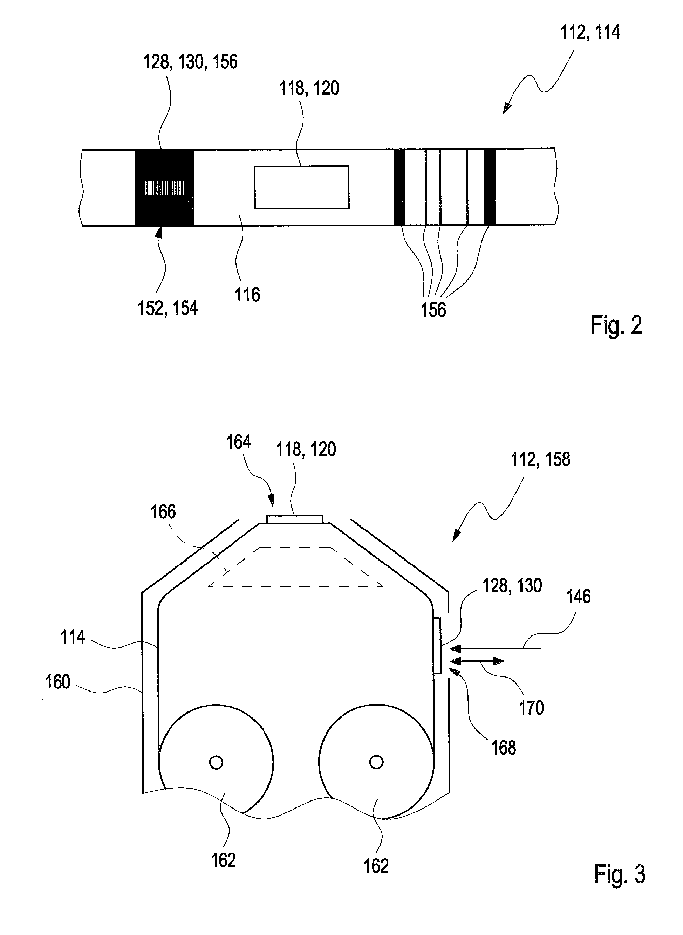

Das analytische Verbrauchsmittel 112 ist in Figur 1 symbolisch in Form eines Analysebandes 114 dargestellt, welches in dem dargestellten Zustand jeweils noch als Halbfertigprodukt oder Zwischenprodukt ausgestaltet ist. Zwischen diesem Halbfertigprodukt und/oder Zwischenprodukt und dem fertigen analytischen Verbrauchsmittel 112 wird begrifflich in dieser Beschreibung nicht unterschieden. Das analytische Verbrauchsmittel 112 kann auch auf andere Weise ausgestaltet sein und kann, zusätzlich zum Analyseband 114, beispielsweise auch weitere Komponenten umfassen, wie unten näher ausgeführt wird. Ein Beispiel einer möglichen Ausgestaltung eines Analysebandes 114 ist in Figur 2 dargestellt und wird unten näher erläutert.

Das Analyseband 114 umfasst einen in diesem Ausführungsbeispiel bandförmig ausgestalteten Träger 116. Dieser Träger 116, welcher als Trägerband ausgestaltet sein kann, kann beispielsweise ein Kunststoff-Trägerband umfassen. Das Verbrauchsmittel 112 umfasst in dem dargestellten Ausführungsbeispiel weiterhin eine Mehrzahl analytischer Hilfsmittel 118, welche in diesem Fall beispielsweise als Testfelder 120 ausgestaltet sein können. Jedes dieser Testfelder 120 kann mindestens eine Nachweischemie umfassen, mit mindestens einem Nachweisreagenz, welches vorzugsweise spezifisch mit einem nachzuweisenden Analyten reagiert und beispielsweise bei Anwesenheit des Analyten eine Farbreaktion durchführt. Es wird darauf hingewiesen, dass die Ausgestaltung des analytischen Hilfsmittels 118 als Testfeld 120 lediglich eine von vielen Möglichkeiten darstellt. Alternativ oder zusätzlich kann das analytische Hilfsmittel 118 beispielsweise auch elektrochemische Testfelder 120 und/oder Lanzetten und/oder andere Arten analytischer Hilfsmittel umfassen. Beispielsweise kann eine Mehrzahl von Lanzetten auf dem Träger 116 vorgesehen sein. Auch eine alternierende Anordnung mehrerer unterschiedlicher Arten analytischer Hilfsmittel 118 ist möglich, beispielsweise eine alternierende Anordnung von Lanzetten und Testfeldern 120.

Entsprechend des analytischen Hilfsmittels 118 umfasst die Vorrichtung 110 mindestens eine Vorrichtung 122 zum Aufbringen des analytischen Hilfsmittels 118. In dem in Figur 1 dargestellten Ausführungsbeispiel ist diese Vorrichtung 122 symbolisch als Etikettiervorrichtung dargestellt, mittels derer die Testfelder 120 in Form von Etiketten auf den bandförmigen Träger 116 aufgebracht werden können. Dabei ist eine Laufrichtung des Bandes bei der Produktion in Figur 1 symbolisch mit der Bezugsziffer 124 bezeichnet. Es wird darauf hingewiesen, dass dies ein Ausführungsbeispiel einer Massenproduktion in einem Rolle-zu-Rolle-Verfahren darstellt. Alternativ oder zusätzlich können jedoch auch andere Arten von Herstellungsverfahren eingesetzt werden, beispielsweise Batch-Verfahren, bei welchen kein kontinuierlicher Träger 116 verwendet wird, sondern bereits ein Träger mit kleineren, endlichen Dimensionen.

Weiterhin umfasst die Vorrichtung 110 eine Aufbringvorrichtung 126. Diese Aufbringvorrichtung 126 ist eingerichtet, um mindestens ein optisch sensitives Material 128 auf den Träger 116 aufzubringen. In dem in Figur 1 dargestellten Ausführungsbeispiel erfolgt dies, indem das optisch sensitive Material 128 in Form von Codierungsfeldern 130 auf den Träger 116 aufgebracht wird. Die Aufbringvorrichtung 126 kann dementsprechend beispielsweise eine Druckvorrichtung umfassen, welche die Codierungsfelder 130 auf den bandförmigen Träger 116 aufdruckt. Dabei können alle gängigen Druckverfahren eingesetzt werden, beispielsweise Tampondruck, Siebdruck, Flexodruck, Tintenstrahldruck, Dispensierverfahren oder Ähnliches. Alternativ oder zusätzlich können auch andere Aufbringtechniken verwendet werden, beispielsweise wiederum Laminiertechniken, analog beispielsweise zur Vorrichtung 122.

Weiterhin umfasst die Vorrichtung 110 in dem in Figur 1 dargestellten Ausführungsbeispiel eine Kalibrationsvorrichtung 132. Diese Kalibrationsvorrichtung 132, welche in Figur 1 symbolisch mit einer Kalibrationslichtquelle 134 und einem Kalibrationsdetektor 136 dargestellt ist, ist eingerichtet, um mindestens eine Eigenschaft des analytischen Verbrauchsmittels 112 zu bestimmen. Beispielsweise kann dies eine Eigenschaft mindestens eines analytischen Hilfsmittels 118, beispielsweise eines oder mehrerer der Testfelder 120, sein. Auf diese Weise können beispielsweise Kalibrationsinformationen generiert werden, beispielsweise Korrekturfaktoren und/oder Funktionskurven, welche in Figur 1 symbolisch mit der Bezugsziffer 138 bezeichnet sind. Diese Kalibrationsinformationen 138 können, wie in Figur 1 angedeutet, beispielsweise an eine Datenverarbeitungsvorrichtung 140 bereitgestellt werden, welche beispielsweise Bestandteil einer unten näher erläuterten Codiervorrichtung 142 sein kann. Auch eine andere Ausgestaltung ist jedoch möglich. Beispielsweise kann die Datenverarbeitungsvorrichtung 140 auch ganz oder teilweise Bestandteil der Kalibrationsvorrichtung 132 sein und/oder es kann eine zentrale Datenverarbeitungsvorrichtung 140 und/oder ein zentraler Datenspeicher genutzt werden. In dem in Figur 1 dargestellten Ausführungsbeispiel wird dabei mittels der Kalibrationsvorrichtung 132 jedes einzelne analytische Hilfsmittel 118 kalibriert. Alternativ könnten diese analytischen Hilfsmittel 118 jedoch auch gruppenweise kalibriert werden, oder es könnte eine gesamte Kalibration für das analytische Verbrauchsmittel 112 und sämtliche darauf enthaltene analytische Hilfsmittel 118 und/oder sämtliche enthaltene Testfelder 120 erfolgen. Verschiedene Ausgestaltungen sind denkbar und für den Fachmann realisierbar.

Weiterhin wird darauf hingewiesen, dass in Figur 1 der Kalibrationsschritt mittels der Kalibrationsvorrichtung 132 noch an einem unfertigen Zwischenprodukt des analytischen Verbrauchsmittels 112 dargestellt ist. Alternativ oder zusätzlich kann der Kalibrationsschritt mittels der Kalibrationsvorrichtung 132 jedoch auch in einem späteren Stadium erfolgen, beispielsweise in einem Stadium, in welchem das Analyseband 114 des analytischen Verbrauchsmittels 112 bereits mit weiteren Komponenten des analytischen Verbrauchsmittels 112 zu einem Fertigprodukt oder einer höheren Zwischenstufe des fertigen analytischen Verbrauchsmittels 112 kombiniert wurde. Dies wird unten anhand der Figur 3 näher erläutert, bei welcher das Analyseband 114 in ein Gehäuse integriert ist.

Weiterhin umfasst die Vorrichtung 110 in dem in Figur 1 dargestellten Ausführungsbeispiel die oben bereits erwähnte Codiervorrichtung 142. Diese Codiervorrichtung 142 kann, neben der optionalen Datenverarbeitungsvorrichtung 140, eine Strahlenquelle 144 zur Erzeugung elektromagnetischer Strahlung 146 sowie gegebenenfalls eine Positioniervorrichtung 148 umfassen. Beides ist in Figur 1 lediglich angedeutet. Die elektromagnetische Strahlenquelle 144 kann beispielsweise einen oder mehrere Laser umfassen. Die Positioniervorrichtung 148 kann beispielsweise eine Scanvorrichtung und/oder eine andere Art von Schreibvorrichtung umfassen, so dass gezielt einzelne Bereiche der Codierungsfelder 130 mit der elektromagnetischen Strahlung 146 beaufschlagt werden können. Auf diese Weise kann ein Einschreiben von Funktionsinformationen in diese Codierungsfelder 130 erfolgen. Alternativ oder zusätzlich kann jedoch auch eine gleichzeitige Belichtung mehrerer, beispielsweise größerer, Bereiche der Codierungsfelder 130 erfolgen, beispielsweise durch Verwendung entsprechender Maskentechniken. Diese Masken können auch flexibel ausgestaltet sein, beispielsweise durch Verwendung von Mikrospiegel-Arrays und/oder sogenannten SLMs (Spatial Light Modulator, räumliche Lichtmodulatoren), so dass auch wechselnde Informationen eingeschrieben werden können. Verschiedene Ausgestaltungen sind denkbar und für den Fachmann realisierbar.

Bei dem in Figur 1 dargestellten Ausführungsbeispiel werden die Kalibrationsinformationen 138 in der Datenverarbeitungsvorrichtung 140, welche alternativ oder zusätzlich auch ganz oder teilweise Bestandteil anderer Komponenten der Vorrichtung 110 sein kann, in Funktionsinformationen umgewandelt. Diese Funktionsinformationen sind in Figur 1 symbolisch mit der Bezugsziffer 150 bezeichnet. Wie oben ausgeführt, wird im Rahmen der vorliegenden Beschreibung begrifflich nicht zwischen den Funktionsinformationen und deren Inhalt und/oder physikalischer Form unterschieden. Diese Funktionsinformationen können somit beispielsweise unmittelbar an die elektromagnetische Strahlenquelle 144 und/oder die Positioniervorrichtung 148 weitergegeben werden. Alternativ oder zusätzlich können jedoch auch entsprechende Steuerbefehle übermittelt werden, beispielsweise Steuerbefehle, welche die Strahlenquelle 144 und/oder die Positioniervorrichtung 148 derart steuern, dass die Funktionsinformationen 150 durch entsprechende lokale Belichtung in einen entsprechenden Code in den Codierungsfeldern 130 umgewandelt werden.

Ein Ausführungsbeispiel eines derartigen Codes 152, welcher mit der Bezugsziffer 150 bezeichnet wird, ist in Figur 2 erkennbar. In diesem Fall ist der Code 152 als strichförmiger Barcode 154 ausgestaltet, welcher in Codierungsfelder 130 eingeschrieben ist. Beispielsweise kann der Barcode 154 in Form eines Farbumschlags in den Codierungsfeldern 130 ausgestaltet sein, beispielsweise indem nicht belichtete Bereiche eine andere Farbe aufweisen als belichtete Bereiche. Wie oben dargestellt, können jedoch auch anders gestaltete optisch detektierbare Veränderungen in den Codierungsfeldern 130 verwendet werden. Alternativ zu einem strichförmigen Barcode 154 können auch andere Arten von Barcodes verwendet werden, beispielsweise zwei- oder dreidimensionale Barcodes.

Die Ausgestaltung des analytischen Verbrauchsmittels 112 gemäß Figur 2 umfasst wiederum, wie oben dargestellt, ein Analyseband 114, auf welches analytische Hilfsmittel 118 in Form von Testfeldern 120 aufgebracht sind. Auch andere Ausgestaltungen sind möglich, wie beispielsweise oben dargestellt wurde.

Zusätzlich zu diesen Testfeldern 120 bzw. analytischen Verbrauchsmitteln 118 umfasst das Analyseband 114 in dem in Figur 2 gezeigten Ausführungsbeispiel eine Mehrzahl von Positionsmarkierungen 156. Diese Positionsmarkierungen, deren Ausgestaltung und Anordnung in Figur 2 lediglich beispielhaft dargestellt ist, ermöglichen einem analytischen Gerät, beispielsweise einem Blutzuckermessgerät, eine exakte Positionierung eines bestimmten Testfeldes 120 vor einem Detektor. Der gesamte Spulvorgang des Analysebandes 114 kann auf diese Weise gesteuert werden.

Bei der in Figur 2 dargestellten Ausführungsform werden diese Positionsmarkierungen 156 ganz oder teilweise mit den Codierungsfeldern 130 zusammengefasst. So kann für alle, einige oder einzelne der Positionsmarkierungen 156 beispielsweise das optisch sensitive Material 128 eingesetzt werden. Beispielsweise kann dieses optisch sensitive Material derart ausgestaltet sein, dass dieses im unbelichteten Zustand dunkel im Vergleich zum Träger 116 ist, beispielsweise schwarz. Durch eine entsprechende Belichtung mit der elektromagnetischen Strahlung 146 kann dann lokal ein Farbumschlag erzielt werden, beispielsweise ein Farbumschlag hin zu einer weißen und/oder hellen Farbe. Der Code 152 kann also als "inverser" Code ausgestaltet sein, mit hellen Codefeldern auf dunklem Hintergrund und/oder als "normaler" Code mit dunklen Codefeldern auf hellem Hintergrund. Alternativ oder zusätzlich sind jedoch auch andere Ausgestaltungen möglich, beispielsweise der Einsatz unterschiedlicher Farben.

Alternativ oder zusätzlich zu der Zusammenfassung der Codierungsfelder 130 mit den Positionsmarkierungen 156 kann jedoch auch eine separate Ausgestaltung der Codierungsfelder 130 erfolgen. So können beispielsweise zusätzlich zu den Positionsmarkierungen 156 räumlich von diesen Positionsmarkierungen 156 getrennte Codierungsfelder 130 auf den Träger 116 aufgebracht werden.

Weiterhin muss nicht notwendigerweise, wie bei dem Ausführungsbeispiel in Figur 2 angedeutet, jedem analytischen Hilfsmittel 118 ein eigenes Codierungsfeld 130 zugeordnet sein. Beispielsweise können auch mehrere analytische Hilfsmittel 118 zusammengefasst werden und dieser Gruppe analytischer Hilfsmittel 118 dann ein gemeinsames Codierungsfeld 130 zugeordnet werde. Auch ist eine Codierung möglich, bei welchem dem gesamten Analyseband 114 lediglich ein einzelnes oder eine Gruppe weniger Codierungsfelder 130 zugeordnet werden, welche dann beispielsweise Funktionsinformationen 150 für das gesamte Analyseband 114 bzw. das gesamte analytische Verbrauchsmittel 112 umfassen können. Verschiedene Ausgestaltungen sind möglich und für den Fachmann im Rahmen der vorliegenden Beschreibung realisierbar.

In Figur 3 ist schließlich ein analytisches Verbrauchsmittel 112 in Form einer Bandkassette 158 schematisch dargestellt. Diese Bandkassette 158 kann ein Gehäuse 160 umfassen, in welchem ein Analyseband 114, beispielsweise gemäß der in Figur 2 beschriebenen Ausgestaltung, aufgenommen sein kann. Dieses Analyseband 114 wird mittels eines oder mehrerer Wickel 162, welche in Figur 3 lediglich angedeutet sind, durch das Gehäuse 160 gespult.

Die Bandkassette 158 umfasst eine in Figur 3 mit der Bezugsziffer 164 bezeichnete Applikationsposition. In dieser Applikationsposition 164 kann beispielsweise eine Applikation einer flüssigen Probe, beispielsweise einer flüssigen Probe einer Körperflüssigkeit (beispielsweise Blut) auf ein Testfeld 120 erfolgen. Weiterhin kann in dieser Applikationsposition 164 auch eine Durchführung der Messung erfolgen. So kann die Bandkassette 158 beispielsweise einen in Figur 3 symbolisch mit der Bezugsziffer 166 bezeichneten Messraum aufweisen, in welchen ein entsprechender Detektor eines analytischen Geräts eingebracht werden kann. Dieser Detektor kann jedoch auch fest in der Bandkassette 158 integriert sein. Mittels dieses in Figur 3 nicht dargestellten Detektors kann beispielsweise eine reflektometrische Messung eines Analyt-bedingten Farbumschlags in dem Testfeld beobachtet werden und anschließend, unter Auswertung dieser Messung, ein qualitativer und/oder quantitativer Rückschluss auf die Anwesenheit eines Analyten, beispielsweise eine Blutglucosekonzentration, vorgenommen werden. Auch andere Messmethoden sind jedoch grundsätzlich denkbar.

Das Gehäuse 160 der Bandkassette 158 umfasst in dem in Figur 3 dargestellten Ausführungsbeispiel weiterhin ein Fenster 168. Dieses Fenster 168 kann beispielsweise als einfache Öffnung in dem Gehäuse 160 ausgestaltet sein. Vorzugsweise, um Verschmutzungen des Analysebandes 114 zu vermeiden, umfasst dieses Fenster 168 jedoch ein Material, beispielsweise einen Kunststoff, welches für elektromagnetische Strahlung 146 transparent ist. Dabei kann eine Transparent für die in der Codiervorrichtung 142 verwendete elektromagnetische Strahlung 146 bestehen und/oder eine Transparenz für ein Detektionslicht 170. Mittels dieses Detektionslichts 170, welches in Figur 3 ebenfalls angedeutet ist, kann beispielsweise die nachweisbare Veränderung der Codierungsfelder 130 im Bereich der Codes 152 ausgelesen werden, beispielsweise durch einfache Barcodeleser. Auch andere Ausgestaltungen sind möglich.

Die in Figur 3 gezeigte Bandkassette 158 bietet den Vorteil, dass diese, bis auf den oben beschriebenen Kalibrationsschritt, zunächst weitgehend fertiggestellt werden kann. Anschließend kann der Kalibrationsschritt, beispielsweise wie anhand Figur 1 beschrieben, durchgeführt werden. In einem sich an den Kalibrationsschritt anschließenden Codierungsschritt können dann mittels der elektromagnetischen Strahlung 146, beispielsweise mittels eines Laserstrahls, durch das Fenster 168 hindurch die Funktionsinformationen 150, welche beispielsweise Kalibrationsinformationen 138 enthalten, in die Codierungsfelder 130 oder einzelne oder eines dieser Codierungsfelder 130 eingeschrieben werden. Während dieses Codierungsvorgangs ist das Analyseband 114 vollständig durch das Gehäuse 160 geschützt, so dass keine Verunreinigungen oder sonstige schädliche Umwelteinflüsse auf das Analyseband 114 einwirken können.

Anschließend kann im Betrieb des analytischen Verbrauchsmittels 112, beispielsweise durch das Fenster 168, der Code 152 wieder ausgelesen werden, um die Funktionsinformationen 150 wieder zu gewinnen. Dieses Auslesen kann beispielsweise mit einem Detektor erfolgen, welcher ohnehin in einem analytischen Gerät vorhanden ist. So kann beispielsweise das Auslesen der Funktionsinformationen 150 in der Applikationsposition 164 selbst erfolgen, beispielsweise mittels desselben Detektors, welcher auch die Testfelder 120 und deren Verfärbungen auswertet. Alternativ oder zusätzlich können auch andere Detektoren eingesetzt werden, beispielsweise Detektoren zur Detektion von Positionsmarkierungen 156. Dies kann beispielsweise ebenfalls wieder in der Applikationsposition 164 erfolgen. Alternativ oder zusätzlich können derartige Detektoren für die Positionsmarkierungen auch angeordnet sein, dass diese im Bereich des Fensters 168 Detektionslicht 170 emittieren bzw. registrieren, um auf diese Weise die Positionsmarkierungen 156 und/oder die Codes 152 zu registrieren. Verschiedene Ausgestaltungen sind denkbar.

### Bezugszeichenliste

- 110: Vorrichtung zur Herstellung eines analytischen Verbrauchsmittels
- 112: Verbrauchsmittel
- 114: Analyseband
- 116: Träger
- 118: analytisches Hilfsmittel
- 120: Testfelder
- 122: Vorrichtung zum Aufbringen des analytischen Hilfsmittels
- 124: Laufrichtung
- 126: Aufbringvorrichtung
- 128: optisch sensitives Material
- 130: Codierungsfelder
- 132: Kalibrationsvorrichtung
- 134: Kalibrationslichtquelle
- 136: Kalibrationsdetektor
- 138: Kalibrationsinformationen
- 140: Datenverarbeitungsvorrichtung
- 142: Codiervorrichtung
- 144: Strahlenquelle
- 146: elektromagnetische Strahlung
- 148: Positioniervorrichtung
- 150: Funktionsinformationen
- 152: Code
- 154: Barcode
- 156: Positionsmarkierungen
- 158: Bandkassette
- 160: Gehäuse
- 162: Wickel
- 164: Applikationsposition
- 166: Messraum
- 168: Fenster
- 170: Detektionslicht

## Patentansprüche

1. Verfahren zur Herstellung eines analytischen Verbrauchsmittels (112), wobei das analytische Verbrauchsmittel (112) mindestens einen Träger (116) und mindestens ein mit dem Träger (116) verbundenes analytisches Hilfsmittel (118) umfasst, wobei mindestens ein optisch sensitives Material (128) auf den Träger (116) aufgebracht wird, wobei das optisch sensitive Material (128) eingerichtet ist, um bei Einwirkung einer elektromagnetischen Strahlung (146) mindestens eine optisch detektierbare Veränderung durchzuführen, wobei in mindestens einem Codierungsschritt mindestens eine Funktionsinformation (150) über das analytische Verbrauchsmittel (112) in das optisch sensitive Material (128) mittels elektromagnetischer Strahlung (146) eingebracht wird, wobei die Funktionsinformation (150) eingerichtet ist, um mindestens einem analytischen Gerät einen korrekten Gebrauch des analytischen Verbrauchsmittels (112) zu ermöglichen.

2. Verfahren nach dem vorhergehenden Anspruch, wobei die elektromagnetische Strahlung (146) eine Laserstrahlung umfasst.

3. Verfahren nach einem der vorhergehenden Ansprüche, wobei in dem Codierungsschritt mindestens ein eindimensionaler und/oder mindestens ein zweidimensionaler und/oder mindestens ein dreidimensionaler Barcode (152, 154) in das optisch sensitive Material (128) eingebracht werden.

4. Verfahren nach einem der vorhergehenden Ansprüche, wobei das optisch sensitive Material (128) in Form mindestens eines Codierungsfeldes (130) auf den Träger (116) aufgebracht wird, insbesondere in Form eines polygonalen und/oder runden Codierungsfeldes (130).

5. Verfahren nach dem vorhergehenden Anspruch, wobei zum Aufbringen des Codierungsfeldes (130) mindestens eines der folgenden Verfahren eingesetzt wird: ein Druckverfahren, bei welchem das optisch sensitive Material (128) und/oder ein Vorstoff des optisch sensitiven Materials (128) auf den Träger (116) aufgedruckt wird; ein Laminierverfahren, bei welchem ein Film des optisch sensitiven Materials (128) auf den Träger (116) auflaminiert und/oder aufgeklebt wird.

6. Verfahren nach einem der beiden vorhergehenden Ansprüche, wobei das analytische Verbrauchsmittel (112) eine Mehrzahl von analytischen Hilfsmitteln (118) umfasst, wobei jedem analytischen Hilfsmittel (118) mindestens ein Codierungsfeld (130) zugeordnet ist.

7. Verfahren nach einem der drei vorhergehenden Ansprüche, wobei Codierungsfelder (130) an mehreren vorgegebenen und bekannten Positionen auf dem analytischen Verbrauchsmittel (112) angeordnet werden, wobei die Codierungsfelder (130) eingerichtet sind, um ganz oder teilweise als Positionsmarkierungen (156) verwendet zu werden.

8. Verfahren nach einem der vorhergehenden Ansprüche, wobei die optisch detektierbare Veränderung eine Veränderung mindestens einer der folgenden physikalischen Eigenschaften des optisch sensitiven Materials (128) umfasst: eine Farbeigenschaft; eine Reflexionseigenschaft; eine Absorptionseigenschaft; einen Brechungsindex.

9. Verfahren nach einem der vorhergehenden Ansprüche, wobei das optisch sensitive Material (128) mindestens eines der folgenden Materialien umfasst: einen Farbstoff, insbesondere einen in einem Matrixmaterial gelösten und/oder dispergierten Farbstoff; ein Pigment, insbesondere; einen Kunststoff, insbesondere einen Kunststoff, welcher bei Einwirkung der elektromagnetischen Strahlung (146) einen Brechungsindex verändert

10. Verfahren nach einem der vorhergehenden Ansprüche, wobei die Funktionsinformation (150) mindestens eine Verbrauchsmittel-spezifische und/oder Hilfsmittel-spezifische Information umfasst, insbesondere mindestens eine der folgenden Informationen: eine Information über einen Hersteller und/oder ein Herstellungsverfahren; eine Information über ein enthaltenes Nachweisreagens; eine Information hinsichtlich eines nachzuweisenden Analyten; eine Information hinsichtlich eines einzusetzenden Analyseverfahrens und/oder Analysesystems; eine Information hinsichtlich der Bedingungen, unter denen eine Analyse durchgeführt werden soll; eine Information hinsichtlich von Parametern und/oder Algorithmen für eine Auswertung von Messungen, insbesondere mindestens einen Korrekturfaktor und/oder mindestens eine Funktionskurve; eine Information hinsichtlich von Chargennummern und/oder mindestens eine Individualkennzeichnung; eine Information hinsichtlich chargenspezifischer Besonderheiten; eine Information hinsichtlich einer Anzahl von analytischen Hilfsmitteln (118); eine Information hinsichtlich einer Art einer Vorrichtung zum Erzeugen und/oder Bereitstellen einer flüssigen Probe, insbesondere einer Lanzette; eine Information hinsichtlich einer zu verwendenden Vorrichtung zum Erzeugen und/oder Bereitstellen einer flüssigen Probe, insbesondere einer Lanzette; ein Haltbarkeitsinformation, insbesondere ein Haltbarkeitsdatum und/oder eine Haltbarkeitsbeschränkung; eine Verwendungsbeschränkung.

11. Verfahren nach einem eher vorhergehenden Ansprüche, wobei das analytische Verbrauchsmittel (112) mindestens eines der folgenden Verbrauchsmittel (112) umfasst: ein analytisches Band (114) mit einem Trägerband und einer Mehrzahl von auf dem Trägerband angeordneten analytischen Hilfsmitteln (118); einen Teststreifen mit mindestens einem analytischen Hilfsmittel (118); eine Testscheibe mit einer Mehrzahl von auf einer Oberfläche und/oder einer Kante der Testscheibe angeordneten analytischen Hilfsmitteln (118); ein faltbares Verbrauchsmittel (112) mit einer Mehrzahl analytischer Hilfsmittel (118).

12. Verfahren nach einem der vorhergehenden Ansprüche, wobei das analytische Hilfsmittel (118) mindestens eines der folgenden analytischen Hilfsmittel (118) umfasst: ein Testfeld (120) zum Nachweis mindestens eines Analyten in einer Probe, insbesondere zum Nachweis eines Metaboliten in einer Körperflüssigkeit; eine Vorrichtung zum Erzeugen und/oder Bereitstellen einer flüssigen Probe, insbesondere eine Lanzette.

13. Verfahren nach einem der vorhergehenden Ansprüche, wobei das analytische Verbrauchsmittel (112) mindestens ein Gehäuse (160) aufweist, wobei das analytische Hilfsmittel (118) zumindest teilweise in dem Gehäuse (160) aufgenommen ist, wobei das Gehäuse (160) mindestens einen transparenten Bereich, insbesondere ein Fenster (168), aufweist, welcher für die elektromagnetische Strahlung (146) und/oder für einen optischen Nachweis der optisch detektierbaren Veränderung zumindest teilweise transparent ist, insbesondere bei einer Wellenlänge der elektromagnetischen Strahlung und/oder einer für den optischen Nachweis der optisch detektierbaren Veränderung verwendeten Wellenlänge.

14. Verfahren nach einem der vorhergehenden Ansprüche, umfassend weiterhin mindestens einen Kalibrationsschritt, wobei in dem Kalibrationsschritt mindestens eine Eigenschaft mindestens eines in dem analytischen Verbrauchsmittel (112) umfassten analytischen Hilfsmittels (118) gemessen wird, wobei diese Eigenschaft zumindest teilweise in die Funktionsinformation (150) oder einen Teil der Funktionsinformation (150) umgewandelt wird, wobei anschließend der Codierungsschritt durchgeführt wird.

15. Vorrichtung (110) zur Herstellung eines analytischen Verbrauchsmittels (112), insbesondere unter Verwendung eines Verfahrens gemäß einem der vorhergehenden Verfahrensansprüche, wobei das analytische Verbrauchsmittel (112) mindestens einen Träger (116) und mindestens ein mit dem Träger (116) verbundenes analytisches Hilfsmittel (118) umfasst, wobei die Vorrichtung (110) mindestens eine Aufbringvorrichtung (126) zum Aufbringen eines optisch sensitiven Materials (128) auf den Träger (116) umfasst, wobei das optisch sensitive Material (128) eingerichtet ist, um bei Einwirkung einer elektromagnetischen Strahlung (146) mindestens eine optisch detektierbare Veränderung durchzuführen, wobei die Vorrichtung (110) weiterhin mindestens eine Codiervorrichtung (142) umfasst, wobei die Codiervorrichtung (142) eingerichtet ist, um mittels elektromagnetischer Strahlung (146) mindestens eine Funktionsinformation (150) über das analytische Verbrauchsmittel (112) in das optisch sensitive Material (128) einzubringen, wobei die Funktionsinformation (150) eingerichtet ist, um mindestens einem analytischen Gerät einen korrekten Gebrauch des analytischen Verbrauchsmittels (112) zu ermöglichen.
